# EUROPÄISCHE PATENTSCHRIFT

(11) **EP 3 927 842 B1**
(45) Veröffentlichungstag und Bekanntmachung des Hinweises auf die Patenterteilung: **30.04.2025**
(21) Anmeldenummer: 20704865.3
(22) Anmeldetag: 12.02.2020
(51) Int. Cl.: C12Q 1/6869, B01L 3/00, B01J 19/00

(54) **VORRICHTUNG ZUR UNTERSUCHUNG EINER BIOLOGISCHEN PROBE**
DEVICE FOR ANALYSING A BIOLOGICAL SAMPLE
DISPOSITIF D'INSPECTION D'UN ÉCHANTILLON BIOLOGIQUE

(30) Priorität: 19.02.2019 DE 102019202174
(43) Veröffentlichungstag der Anmeldung: 29.12.2021
(73) Patentinhaber: Robert Bosch GmbH, 70442 Stuttgart (DE)
(72) Erfinder: HOFFMANN, Jochen, 71272 Renningen (DE)
(86) Internationale Anmeldenummer: PCT/EP2020/053511
(87) Internationale Veröffentlichungsnummer: WO 2020/169422

(56) Entgegenhaltungen:
- WO-A1-2010/100265
- WO-A1-2015/002975
- WO-A2-2011/002957
- US-A1- 2007 243 634
- US-A1- 2018 355 350

## Beschreibung

### Stand der Technik

Hier beschrieben wird eine Vorrichtung zur Untersuchung einer biologischen Probe, wobei es sich bei der Vorrichtung insbesondere um ein fluidisches Netzwerk zur parallelen Durchführung einer Vielzahl von Untersuchungen von biologischen Proben handelt. Die biologische Probe ist insbesondere eine Probe mit darin enthaltenen Nukleinsäuresequenzen. Die Untersuchung, die mit der Vorrichtung durchgeführt wird, zielt insbesondere auf die Identifikation einer Nukleinsäuresequenz bzw. darauf ab, festzustellen, ob eine bestimmte Nukleinsäuresequenz in einer Probe enthalten ist (oder nicht). Solche Untersuchungen sind insbesondere für die Entschlüsselung von genetischen Codes (DNA-Sequenzierung) notwendig.

Die Entschlüsselung des genetischen Codes (DNA-Sequenzierung) ist ein Standard-Verfahren in Forschung und Medizin.

Eine besondere Form von solchen Analysen ist das sogenannte Next Generation Sequencing (NGS). Mit dem Begriff "Next Generation Sequencing" werden eine ganze Reihe von verschiedenen Verfahren zur Sequenzierung bezeichnet, welche sich alle im Wesentlichen dadurch auszeichnen, dass automatisiert eine größere Menge von Untersuchungen in kurzer Zeit (insbesondere auch parallel) durchgeführt werden können. Bei den zum "Next Generation Sequencing" zählenden Verfahren kommen hauptsächlich Sequencing-by-Synthesis-Verfahren zum Einsatz.

Bei diesen Verfahren werden die zu sequenzierenden DNA-Moleküle zunächst vervielfältigt (klonal amplifiziert) und auf einer Festphase angebunden. Die Erkennung bestimmter Nukleinsäuresequenzen erfolgt mit Hilfe von Markern (fluoreszenzgelabelten und terminierten Nukleotiden), die an den DNA-Molekülen an Stellen anlagern, an denen bestimmte Muster existieren. Bei der Synthese des Produktstranges wird der Einbau einzelner fluoreszenzmarkierter Nukleotide an der DNA gemessen, was schlussendlich auf die Sequenz rückschließen lässt. Die in diesen Systemen durchgeführten Verfahren werden auch als Sequencing-by-Synthesis (SBS) bezeichnet.

Das Ablesen der Sequenz erfolgt beim SBS in der Regel durch die zyklische Durchführung der folgenden Schritte:
1) Beaufschlagung (= Einspülen + Inkubation) des Reaktionsbereiches mit fluoreszenz gelabelten und terminierten Nukleotiden.
2) Einbau der Nukleotide.
3) Waschen/Spülen des Reaktionsbereiches.
4) Optische Detektion von Fluoreszenzereignissen, und Erkennen, wo die Nukleotide eingebaut wurden,
5) Beaufschlagung (= Einspülen + Inkubation) des Reaktionsbereiches mit einer Lösung für die Entfernung der terminierten Nukleotide.
6) Waschen/Spülen des Reaktionsbereiches.

Eine Herausforderung für die Miniaturisierung eines solchen Sequenzierverfahrens sind die Vielzahl an Wasch- und Spülschritten die durchgeführt werden, um dem Reaktionsbereich die Vielzahl an wechselnder Fluide zuzuführen. Kommerzielle Sequenzierer benötigen beispielsweise bis zu 100 mL [Milliliter] Waschlösung pro Sequenzierung.

Die US 2007/243634 A1 beschreibt eine mikrofluidische Vorrichtung zur Durchführung eines "Droplet-basierenden" Verfahrens, beispielsweise zur Vervielfältigung von Nukleinsäuren oder zur Analyse der Sequenzen von Nukleinsäuren.

Die WO 2011/002957 A2 beschreibt eine mikrofluidische Vorrichtung zur Durchführung eines "Droplet-basierenden" Verfahrens, um derartige molekulare Techniken zu erleichtern.

Die US 2018/355350 A1 offenbart ein Verfahren und eine Vorrichtung zur Durchführung einer Sequenzierung von immobilisierten Nukleinsäuren in einem "Dropletformat".

### Offenbarung der Erfindung

Hier beschrieben werden soll eine Vorrichtung, mit der eine Analyse von biologischen Proben (insbesondere DNA-Proben) nach dem Ansatz des Sequencing-by-Synthesis effizient durchgeführt werden kann. Mit der beschriebenen Vorrichtung wird es insbesondere möglich, die für eine Sequenzierung benötigten Volumina der einzelnen Reagenzien (Reagenzien mit Nukleotiden, Spüllösungen etc.,) drastisch zu reduzieren.

Die hier beschriebene Vorrichtung ist ein fluidisches Netzwerk zur parallelen Durchführung einer Vielzahl von Untersuchungen von biologischen Proben, aufweisend ein Durchflussarray mit einer Vielzahl von Reaktionsräumen, wobei die Reaktionsräume jeweils einen ersten Kanalanschluss und einen zweiten Kanalanschluss aufweisen, wobei der erste Kanalanschluss an einen ersten Versorgungskanal angeschlossen ist und der zweite Kanalanschluss an einen zweiten Versorgungskanal angeschlossen ist, wobei der erste Versorgungskanal und der zweite Kanalanschluss mit einer Zirkulationsleitung miteinander verbunden sind, wobei an der Zirkulationsleitung mindestens eine Komponente angeschlossen ist, mit welcher Versuchsreagenzien in die Reaktionsräume des Durchflussarray einbringbar sind.

Kern des beschriebenen fluidischen Netzwerks ist das Durchflussarray. In dem Durchflussarray sind die Reaktionsräume enthalten, in welchen die Untersuchungen durchgeführt werden können, für welche das beschriebene fluidische Netzwerk vorgesehen ist. Das Durchflussarray hat bevorzugt eine Vielzahl von Reaktionsräumen - beispielsweise mehr als 100 Reaktionsräume, mehr als 200 Reaktionsräume oder sogar mehr als 1000 Reaktionsräume.

Bei dem fluidischen Netzwerk handelt es sich insbesondere um ein mikrofluidisches Netzwerk. Der Begriff "mikrofluidisch" bezieht sich hier vor allem auf die Größenordnung der mikrofluidischen Vorrichtung. Die mikrofluidische Vorrichtung ist in diesem Zusammenhang insbesondere dadurch gekennzeichnet, dass in den darin angeordneten fluidischen Kanälen und Kammern physikalische Phänomene relevant sind, die im Allgemeinen der Mikrotechnik zugeordnet werden. Hierzu zählen beispielsweise Kapillareffekte, Effekte (insbesondere mechanische Effekte) die im Zusammenhang mit Oberflächenspannungen des Fluids stehen. Hinzu zählen weiterhin Effekte wie Thermophorese und Elektrophorese. Diese Phänomene sind in der Mikrofluidik üblicherweise dominant gegenüber Effekten wie der Schwerkraft. Die mikrofluidische Vorrichtung kann auch dadurch gekennzeichnet sein, dass sie zumindest teilweise mit einem schichtweisen Verfahren hergestellt ist und Kanäle zwischen Schichten des Schichtaufbaus angeordnet sind. Der Begriff "mikrofluidisch" kann auch über die Querschnitte innerhalb der Vorrichtung charakterisiert werden, welche zur Führung des Fluids dienen. Üblich sind beispielsweise Querschnitte im Bereich von 10 µm [Mikrometer] mal 10 µm bis hin zu 800 µm mal 800 µm.

Vorzugsweise ist das fluidische Netzwerk in der Art eines Lab-on-Chip gebildet. In diesem Zusammenhang kann das fluidische Netzwerk beispielsweise Teil eines Lab-on-Chips, beispielsweise in und/oder auf dem Chip angeordnet sein. Weiterhin kann das fluidische Netzwerk in und/oder auf dem Chip integriert sein.

Mit dem Begriff "Kanalanschluss" sind Anschlüsse der Reaktionsräume an sogenannte Versorgungskanäle gemeint, welche als eine Art Verteiler dienen, um Fluid aus der Zirkulationsleitung in die einzelnen Reaktionsräume des Durchflussarrays zu verteilen. Insbesondere sind die ersten Kanalanschlüsse an einen gemeinsamen ersten Versorgungskanal angeschlossen und/oder die zweiten Kanalanschlüsse an einen gemeinsamen zweiten Versorgungskanal angeschlossen.

Die Zirkulationsleitung dient der zentralen Steuerung der Einbringung von Reagenzien in die Reaktionsräume. Flüssigkeitsplugs (Flüssigkeitsabschnitte, getrennt durch ein Fördermedium, wie beispielsweise Öl) in der Zirkulationsleitung können durch eine Förderung in der Zirkulationsleitung über die Versorgungskanäle und die Kanalanschlüsse in die Reaktionskammern eingebracht werden.

Mit dem Begriff "Komponente" sind sämtliche Komponenten gemeint, die der Einbringung von Reagenzien in das Durchflussarray bzw. der Steuerung dieser Einbringung dienen, beispielsweise Fördereinrichtungen, Filter, Reservoire, Ventile

Besonders bevorzugt ist, wenn das fluidische Netzwerk zur Untersuchung einer biologischen DNA-Probe und zur Erkennung von Nukleinsäuresequenzen in der DNA-Probe eingerichtet ist.

Außerdem bevorzugt ist das fluidische Netzwerk, wenn an die Zirkulationsleitung mindestens eine Fördereinrichtung angeschlossen ist, mit welcher Reagenzien über den ersten Versorgungskanal und/oder über den zweiten Versorgungskanal in die Reaktionsräume förderbar sind.

Die Fördereinrichtung ist beispielsweise eine Kolben- oder Membranpumpe. Besonders bevorzugt hat die Fördereinrichtung eine gezielt umkehrbare Förderrichtung, um eine Förderung in der Zirkulationsleitung in beide Richtungen zu ermöglichen und um damit beeinflussen zu können, ob und wenn ja, wann und wie Flüssigkeitsplugs mit bestimmten Reagenzien in die Reaktionskammern des Durchflussarrays gelangen.

Weiter bevorzugt ist das fluidische Netzwerk, wenn an die Zirkulationsleitung mindestens ein Reservoir angeschlossen ist, in welchem ein Reagenz bereitgestellt wird.

Besonders bevorzugt sind an die Zirkulationsleitung eine Vielzahl von derartigen Reservoirs angeschlossen. Besonders bevorzugt existieren einerseits Reservoirs für Reagenzien, die in dem Untersuchungsprozess, welcher mit dem beschriebenen fluidischen Netzwerk durchführbar ist, eine aktive Rolle spielen (sogenannte Reaktionsreagenzien). Besonders bevorzugt existieren zusätzlich Reservoirs für weitere Fluide, die lediglich eine mechanische bzw. fluidmechanische Wirkung in dem Betrieb des fluidischen Netzwerks haben (wie beispielsweise zwischen den Plugs vorgesehenes Fördermedium). Hierzu zählen beispielsweise Fluide, die für die im Rahmen des Betriebs des beschriebenen fluidischen Netzwerks durchgeführten Reaktionen im Wesentlichen inert sind. Solche Fluide dienen insbesondere dazu, Reaktionsreagenzien voneinander zu trennen. Solche Fluide sind insbesondere Öle.

Besonders bevorzugt ist das fluidische Netzwerk, wenn an die Zirkulationsleitung mindestens ein Reservoir angeschlossen ist, in welchem ein Verdrängermedium bereitgestellt wird.

Mit einem Verdrängermedium kann gezielt Flüssigkeit verdrängt werden. Insbesondere können Flüssigkeiten verdrängt werden, die im Rahmen von mit der beschriebenen Vorrichtung durchgeführten Analyseverfahren mit anderen Reagenzien und/oder Proben interagieren. So kann eine Trennung von Reagenzien zueinander und/oder von Proben und Reagenzien voneinander erreicht werden.

Das Verdrängermedium kann ein Mineralöl, Silikonöl, ein fluoriertes Öl oder Derivate oder Mischformen genannter Öle sein.

Besonders bevorzugt sind solche Reservoire so an die Zirkulationsleitung anbindbar, dass diese jeweils gezielt aktiv zugeschaltet oder deaktiviert werden können, so dass Fluide aus einzelnen Reservoiren gezielt in die Zirkulationsleitung gelangen können und somit auch gezielt den Reaktionsräumen in dem Durchflussarray zuführbar sind.

Darüber hinaus bevorzugt ist das fluidische Netzwerk, wenn mindestens ein Reservoir mit einem Ventil der Zirkulationsleitung zuschaltbar ist.

Es können auch mehrere Ventile vorgesehen sein, um Reservoire (gezielt) zuzuschalten. Beispielsweise können Ventilanordnungen ausgeführt sein, mit welchen die Zirkulationsleitung wahlweise an dem jeweiligen Reservoir vorbei oder durch das jeweilige Reservoir hindurch geschaltet werden kann, um Reagenzien aus dem jeweiligen Reservoir in die Zirkulationsleitung und damit auch für die Prozesse innerhalb der Reaktionskammern bereitzustellen.

Außerdem bevorzugt ist das fluidische Netzwerk, wenn in der Zirkulationsleitung mindestens ein Filter angeordnet ist, mit welchem ein in der Zirkulationsleitung bewegtes Reagenz filterbar (filtrierbar) ist.

Ein solcher Filter ist gegebenenfalls auch über Ventile der Zirkulationsleitung optional zuschaltbar. Er dient zum Reinigen der Fluide, die in der Zirkulationsleitung gefördert werden.

Darüber hinaus ist das fluidische Netzwerk vorteilhaft, wenn die Reaktionsräume in dem Durchflussarray in (bzw. zu) einer zweidimensionalen Matrix angeordnet sind. Dies bedeutet mit anderen Worten insbesondere, dass die Reaktionsräume so relativ zueinander angeordnet sind, dass sie gemeinsam eine (zweidimensionale) Matrix bilden.

Eine solche Anordnung ermöglicht es, Reaktionsräume auf dem Durchflussarray kompakt und platzsparend bereit zu stellen.

Weiter bevorzugt ist das fluidische Netzwerk, wenn die Reaktionsräume in dem Durchflussarray mit Trennstegen voneinander abgetrennt sind.

Das Durchflussarray kann besonders bevorzugt als eine Art Lochblech ausgeführt sein, bei welchem die einzelnen Löcher die Reaktionskammern bilden und das dazwischen verbliebene Material die Trennstege ausbildet.

Außerdem bevorzugt ist das fluidische Netzwerk, wenn in den Reaktionsräumen Rückhaltenasen vorgesehen sind, mit welchen Partikel in den Reaktionsräumen gehalten werden können.

Darüber hinaus ist das fluidische Netzwerk bevorzugt, wenn auf einer ersten Seite des Durchflussarrays ein Einkopplungsbereich zum Einbringen einer Anregung mit einer Anregungseinheit und auf einer zweiten Seite des Durchflussarrays ein Auslesebereich zum optischen Auslesen des Durchflussarrays mit einer Detektionseinheit angeordnet sind. Der Einkopplungsbereich ist in diesem Zusammenhang in der Regel zum Einbringen einer Anregung mit einer Anregungseinheit eingerichtet. Der Auslesebereich ist in diesem Zusammenhang in der Regel zum optischen Auslesen des Durchflussarrays mit einer Detektionseinheit eingerichtet. Ist gibt auch optische Verfahren, bei denen Anregungslicht und Auslese der emittierten Fluoreszenzsignale von der gleichen Seite aus erfolgen. Wenn solche Verfahren zur Anwendung kommen ist es auch möglich, dass der Einkopplungsbereich und der Auslesebereich gemeinsam auf einer Seite (auf der ersten Seite oder der zweiten Seite des Durchflussarrays ausgebildet sind.

Die erste Seite und die zweite Seite (und damit auch der Einkopplungsbereich und der Auslesebereich) sind auf (einander) gegenüberliegenden, flächigen Seiten des Durchflussarrays angeordnet und befinden sich bevorzugt jeweils auf der dem Durchflussarray abgewandten Seite des ersten Versorgungskanals bzw. des zweiten Versorgungskanals. Die Anregungseinheit ist bevorzugt dazu eingerichtet (bspw. durch Strahlung), optische Marker in den Reaktionsräumen des Durchflussarrays anzuregen, so dass diese ein optisches Signal abgeben. Die Detektionseinheit (beispielsweise eine Kamera) ist bevorzugt dazu eingerichtet, solche optischen Signale zu detektieren und damit auch eine Auswertung zu ermöglichen.

Hier beschrieben werden soll auch ein fluidisches Netzwerk, wobei das Durchflussarray als austauschbares Einlegeteil ausgeführt ist. Das Durchflussarray kann in diesem Zusammenhang beispielsweise in der Art eines wechselbaren Moduls gebildet sein. Insbesondere ist das Durchflussarray mit der restlichen beschriebenen Vorrichtung (dem restlichen fluidischen Netzwerk) lösbar verbindbar. In diesem Zusammenhang ist es besonders vorteilhaft, wenn das Einlegeteil mit einem Material gebildet ist, welches sich von zumindest einem Material der restlichen Vorrichtung unterscheidet.

Das Durchflussarray kann insbesondere als Kartusche ausgeführt sein, welche in die restliche beschriebene Vorrichtung (das beschriebene fluidische Netzwerk) austauschbar einsetzbar ist. Durch das Einsetzen des Durchflussarrays werden die Reaktionsräume des Durchflussarrays mit den Versorgungskanälen und damit auch mit der Zirkulationsleitung verbunden.

Besonders bevorzugt ist das fluidische Netzwerk, wenn ein Durchmesser von Reaktionsräumen in dem Durchflussarray zwischen 1 nm [Nanometer] und 500 µm [Mikrometer], bevorzugter Weise zwischen 1 µm und 50 µm, beträgt.

Reaktionsräume in dieser Größenordnung haben sich für die beschriebene Vorrichtung (das beschriebene fluidische Netzwerk) und die damit durchführbaren Versuche als besonders vorteilhaft erwiesen.

Kern der beschriebenen Vorrichtung ist das Durchflussarray, welches man sich insbesondere vorstellen kann wie ein miniaturisiertes Lochblech. Die einzelnen Löcher dieses Lochbleches sind die Reaktionskammern, in denen die einzelnen Erkennungsschritte stattfinden können. Bevorzugt sind in den Reaktionskammern jeweils verschiedene Nukleotiden angeordnet, an denen die zu sequenzierenden DNA-Moleküle, welche über die Versorgungskanäle in die Reaktionskammern gelangen können, immobilisiert sind. Weiterhin können die zu sequenzierenden DNA-Sequenzen auch durch einen im Stand der Technik bekannten Prozess namens BEAMing hergestellt werden. Die hierbei entstehenden Beads, auf deren Oberflächen eine Vielzahl DNA-Stränge gleicher Sequenz angebunden sind, können in einem nächsten Schritt in das Durchlocharray eingespült und dort zurückgehalten (=fixiert) werden. Durch die Kombination aus einer in Wasser unlöslichen, nicht mischbaren Phase (z.B. Mineralöl) mit den wässrigen Prozessflüssigkeiten kann in einem solchen Durchflussarray eine vorteilhafte Sequenzierung nach folgendem Grundprinzip erreicht werden: In einem ersten Schritt können die Durchlöcher mit der Flüssigkeit des jeweiligen Schrittes beaufschlagt/befüllt werden, was über die Versorgungskanäle geschieht. Im nächsten Schritt können Ober- und Unterseite des Durchlocharrays durch Über- und/oder Unterschichtung mit Öl versiegelt werden, was ebenfalls über die Versorgungskanäle geschehen kann.

In diesem fluidischen Zustand (wenn die Reaktionskammern verschlossen sind) können die jeweiligen Prozessschritte des "Sequencing-by-senthesis" durchgeführt werden. Vor Durchführung des nächsten Prozesschrittes des "Sequencing-by-senthesis" können nun die Flüssigkeiten in den Reaktionskammern des vorigen Schrittes mittels Durchspülung mit Öl direkt verdrängt werden. Alternativ oder zusätzlich kann mit Wasser-in-Öl Plugs gearbeitet werden, wodurch es möglich ist, eine kleine Menge an Waschlösung (bereitgestellt und befördert als Plug) durch die Durchlöcher zu spülen.

Hier auch beschrieben werden soll ein Verfahren zur Durchführung einer Analyse einer biologischen Probe aufweisend die folgenden Schritte:
a) Bereitstellen eines fluidischen Netzwerks nach einem der vorstehenden Ansprüche,
b) Bereitstellen mindestens einer Probe in einem Durchflussarray des fluidischen Netzwerks.
c) Bereitstellen von mindestens zwei verschiedenen Reagenzien in dem fluidischen Netzwerk, wobei die beiden verschiedenen Reagenzien durch ein Verdrängermedium voneinander getrennt sind.
d) Beaufschlagen der Probe mit den mindestens zwei verschiedenen Reagenzien, wobei ein direkter Kontakt der Reagenzien durch das Verdrängermedium verhindert wird.

Die Probe kann in Schritt b) beispielsweise zusammen mit dem Durchflussarray in das fluidische Netzwerk eingesetzt werden. Dies gilt insbesondere, wenn die Probe nach Art einer Kartusche ausgeführt ist.

Das Trennen verschiedener Reagenzien in Schritt c) erfolgt bevorzugt dadurch, dass in einer Zirkulationsleitung der beschriebenen Vorrichtung Plugs aus Verdrängermedium gebildet sind, die verschiedene Reagenzien voneinander abtrennen.

Das getrennte Beaufschlagen der Probe mit den verschiedenen Reagenzien in Schritt d) erfolgt bevorzugt dadurch, dass mit Hilfe des Verdrängermediums ein erstes Reagenz bevorzugt vollständig aus dem Bereich der Probe verdrängt bzw. von der Probe entfernt wird bevor die Probe einem zweiten Reagenz ausgesetzt wird.

Das beschriebene fluidische Netzwerk sowie das beschriebene Verfahren und insbesondere die Kombination aus einem Durchflussarray als Sequenzierbereich, der Verwendung von Wasser-in-Öl-Gemischen und der Miniaturisierung des Arrays sind besonders vorteilhaft. Hierdurch werden insbesondere einer oder mehrere der folgenden Vorteile ermöglicht:
- Durch die beschriebene Vorrichtung (das beschriebene fluidische Netzwerk) lassen sich sämtliche Prozessflüssigkeiten eines Sequencing-by-Synthesis-Verfahrens reduzieren. Dies ist ein großer Vorteil für eine Lab-on-Chip basierte Sequenzierung, bei der nur begrenzte Mengen an Reaktionslösungen vorlagerbar sind.
- Es werden nicht nur die Mengen an benötigten Waschreagenzien reduziert, sondern auch benötigte Mengen an Reaktionslösungen, die teure Enzyme enthalten. Dies ermöglicht eine starke Kostenreduktion.
- Das Durchflussarray ermöglicht es, die für die optische Auslese einer Sequenzierreaktion benötigte Anregungs- und Detektionseinheit auf zwei verschiedenen Seiten des Durchlocharrays zu platzieren, was zu einer verbesserten optischen Effizienz und mehr Anordnungsmöglichkeiten führt.
- Das Durchflussarray kann als Einlegeteil (wie ein DNA-Mikroarray) gestaltet werden, was Freiheiten bei der Materialwahl und Vorteile beim Aufbauprozess einer des Durchflussarrays bedeutet.
- Mit dem beschriebenen fluidischen Netzwerk ist ein Sample-to-sequence-Prozess möglich. Dies hat das enorme Potential, DNA-Signaturen oder sogar SNPs zu erkennen, die nicht von einer auf dem Chip vorhandenen Sonde erkannt werden können. Aufgrund von immer auftretenden Mutationen in dem Ziel-Genom ist eine Workflow für die Sequenzierung ein Schlüssel zur Zukunftssicherheit eines Lab-on-Chip-Systems.
- Mit Hilfe des beschriebenen fluidischen Netzwerks besteht die Möglichkeit, die Sequenzierreaktion so lange laufen zu lassen, bis Gensignaturen eindeutig bestimmt sind. Es ist beispielsweise auch möglich, nur ein einziges Nukleotid einzubauen, wenn es alleinig um den Nachweis von SNPs geht.

Die beschriebene Vorrichtung wird nachfolgend anhand der Figuren näher erläutert. Die Figuren zeigen nur ein bevorzugtes Ausführungsbeispiel, auf welches die Offenbarung der beschriebenen Vorrichtung nicht begrenzt ist. Es zeigen:
- Fig. 1:: ein beschriebenes fluidisches Netzwerk
- Fig. 2:: einen Ausschnitt aus dem fluidischen Netzwerk gemäß Fig. 1,
- Fig. 3:: eine erste Ausführungsvariante eines Durchflussarrays,
- Fig. 4:: eine zweite Ausführungsvariante eines Durchflussarrays,
- Fig. 5:: eine dritte Ausführungsvariante eines Durchflussarrays,
- Fig. 6:: eine vierte Ausführungsvariante eines Durchflussarrays, und
- Fig. 7:: eine Veranschaulichung von Vorgängen eines mittels der beschriebenen Vorrichtung durchführbaren Verfahrens.

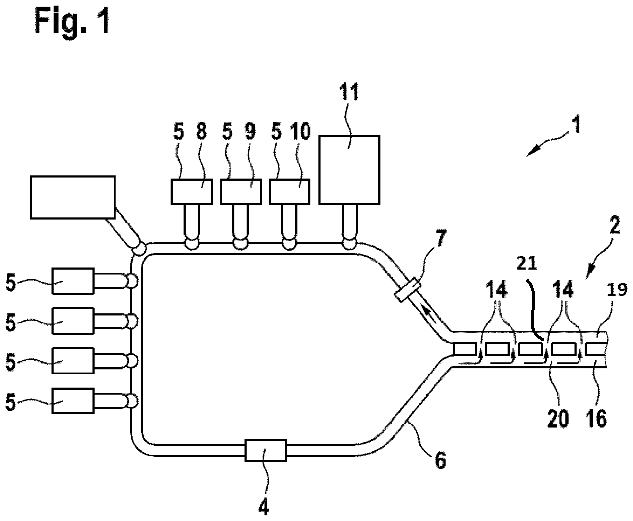

In Fig. 1 ist ein beispielhaftes beschriebenes fluidisches Netzwerk 1 für eine Sequenzierung gezeigt. Das mikrofluidische Netzwerk 1 umfasst bevorzugt eine Zirkulationsleitung 6 und ein Durchflussarray 2 mit Reaktionsräumen 14. Die Reaktionsräume 14 bilden Durchflusszellen, durch die ein in der Zirkulationsleitung 6 zirkulierendes Fluid parallel strömt. In den Reaktionsräumen befinden sich bevorzugt Kolonien immobilisierter DNA-Moleküle. In jedem einzelnen Reaktionsraum 14 kann eine Sequenzierreaktion (=Entschlüsselung der Basenabfolge) stattfinden. Die dafür vorgesehenen Reagenzien fließen beispielsweise von einer ersten Seite 22 ausgehend durch einen ersten Versorgungskanal 16 in erste Kanalanschlüsse 20 der Reaktionskammern 14. Bevorzugt ist die erste Seite 22 eine Unterseite des Durchflussarrays 2. Das heißt, dass das Durchflussarray 2 bevorzugt (geodätisch) so ausgerichtet ist, dass es von unten nach oben durchströmt wird. Aus der Reaktionskammer 14 treten die Reagenzien an zweiten Kanalanschlüssen 21 an einer zweiten Seite 24 des Durchflussarrays 2 aus, um dann in einen zweiten Versorgungskanal 19 einzutreten. Der erste Versorgungskanal 16 bildet bevorzugt also einen Einströmkanal in die Reaktionskammern 14. Der zweite Versorgungskanal 19 bildet bevorzugt also einen Ausströmkanal aus den Reaktionskammern 14 hinaus. Der erste Versorgungskanal 16 und der zweite Versorgungskanal 19 sind über die Zirkulationsleitung 6 miteinander verbunden.

Das Durchflussarray 2 stellt eine Vielzahl von parallel zueinander angeordneten Reaktionsräumen 14 bereit. An dem mikrofluidischen Netzwerk 1 sind, abgetrennt über Ventile 3, verschiedene Reservoire 5 angeschlossen. Diese Reservoire 5 halten die verschiedenen, für eine Sequenzierreaktion vorgesehenen Reagenzien bereit. Für eine Sequencing-by-Synthesis-Analyse werden beispielsweise gelabelte Nukleotide (bspw. ddNTPs; A = Adenin, G = Guanin, T = Thymin, C = Cytosin) eingesetzt, die von einer Polymerase (z.B. "Enzym1") in die abzulesende Nukleinsäuresequenz eingebaut werden. An den eingebauten Nukleotiden ist ein Terminator befestigt, der den Einbau eines weiteren Nukleotids verhindert. In einer Sequenzierreaktion wird vor der Zugabe eines weiteren Nukleotids dieser Terminator entfernt, beispielsweise durch ein "Enzym2". Weitere Reagenzien sind der Sequenzierprimer, der den Startpunkt der Sequenzierung definiert. In weiteren Reservoir(en) sind Waschpuffer vorhanden, mittels denen die in dem Durchflussarray 2 befindlichen Reaktionslösungen vor einem nächsten (Reaktions-)Schritt weggespült werden können.

Optional enthält das Netzwerk einen Filter 7. Mit dem Filter 7 können Bestandteile, die der Waschpuffer aufgenommen hat, wieder entfernt werden. Der Filter 7 kann eine dielektrophoretische Einheit umfassen, durch die die filtrierende Wirkung erreicht wird und mit der einer Lösung z.B. Nukleotide entzogen werden können. Damit wird eine "Waschpufferrückgewinnung" zur vorteilhaften Reduktion an vorgesehenem Waschpuffer ermöglicht. In dem Netzwerk befindet sich mindestens eine Fördereinrichtung 4, welche bspw. eine Membranpumpe oder eine peristaltische Pumpe sein kann. Das fluidische Netzwerk 1 weist außerdem ein Abfallreservoir 11 auf, um nicht mehr verwendete Reagenzien aufzunehmen. In einem (nicht gezeigten) Reservoir ist ein Verdrängermedium gelagert, was zur physikalischen Trennung der Reagenzien oder zur direkten Verdrängung von in dem Durchflussarray 2 befindlichen Reagenzien (also eine Art Waschunterstützung) verwendet werden kann. Das Verdrängermedium kann ein Mineralöl, Silikonöl, ein fluoriertes Öl oder Derivate oder Mischformen genannter Öle sein.

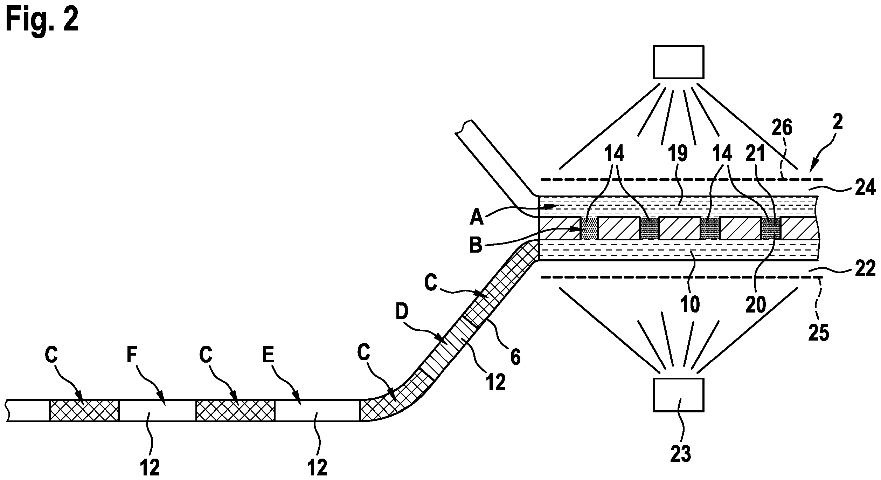

In Fig. 2 ist ein beispielhafter fluidischer Ausschnitt einer Sequenzierung in einem beschriebenen fluidischen Netzwerk 1 zu sehen. Schwerpunktmäßig zeigt die Fig. 2 das beschriebene Durchflussarray 2 des fluidischen Netzwerks 1. An dem Durchflussarray sind die bereits beschriebenen Reaktionskammern 14 mit den ersten Kanalanschlüssen 20 und den zweiten Kanalanschlüssen 21 zu erkennen sowie der erste Versorgungskanal 16 auf der ersten Seite 22 und der zweite Versorgungskanal 19 auf der zweiten Seite 24 des Durchflussarrays. Auf der ersten Seite 22 ist ein Einkopplungsbereich 25 angeordnet, an welchem eine Anregungseinheit 23 eine Anregung in die Reaktionsräume 14 geben kann. So können Moleküle in den Reaktionsräumen 14 angeregt werden, damit diese optische Signale aussenden. Solche optische Signale können an einem Auslesebereich 26 auf der zweiten Seite 24 mit einer Detektionseinheit 27 detektiert werden.

In einem in Fig. 2 dargestellten Abschnitt der Zirkulationsleitung 6 sind mehrere Phasen von Reagenzien zu sehen, die mit Buchstaben bezeichnet sind. Hier zu erkennen sind beispielhaft wässrige Phasen (A,D,E,F) voneinander durch mindestens eine Ölphase C (einem Verdrängermedium) getrennt. Einzelne Phasen (A,D,E,F) bilden in der Zirkulationsleitung 6 Flüssigkeitsplugs 12, die jeweils durch Phasengrenzen 13 voneinander getrennt sind und die dem Durchflussarray 2 bzw. den in dem Durchflussarray enthaltenen Reaktionsräumen 14 mit Hilfe einer Fördereinrichtung in der Zirkulationsleitung 6 (siehe Fig. 1) gezielt zugeführt werden können. Dies geschieht, in dem die Fördereinrichtung fördert bis die jeweiligen Flüssigkeitsplugs 12 in den Reaktionsräumen 14 sind. Um die Verwendung der Fördereinrichtung zum zielgenauen Fördern der Flüssigkeitsplugs 12 in die Reaktionsräume 14 zu unterstützen, können auch die weiter vorne beschriebene Anregungseinheit 23 und die weiter vorne beschriebene Detektionseinheit 27 verwendet werden.

Vor oder nach der Ölphase (oder zwei nacheinander geschalteten verschiedenen Phasen), oder in der Mitte zweier Ölphasen kann zusätzlich noch ein Waschpuffer (allgemein: eine wässrige Phase) vorhanden sein. An den Wänden der Zellen des Durchflussarrays 2 sind jeweils DNA-Kolonien B immobilisiert (wie und woher die kommen, wird noch später erklärt). In der Abbildung sind die Zellen (und der Bauraum drumherum) mit Lösung A befüllt. In diesem Beispiel ist das z.B. die Anbindung eines Sequenzierprimers. Der mit B in Kontakt stehende Plug C ist eine Kombination aus Waschpuffer und Verdrängermedium. Mit diesem Waschschritt werden die Reagenzien aus B aus den Zellen entfernt. Das Mehrphasensystem wird durch die Fördereinheit nun so lange transportiert, bis Plug D die Zellen des Arrays vollständig befüllt. Plug D ist in diesem zusammenhängenden Beispiel ein Gemisch aus Enzym1 und ddNTP "A". Wird das Array mit Plug D beaufschlagt, baut das Enzym (eine Polymerase) bei einem in dem abzulesenden Strang vorhandenen "T" (wegen der Basenpaarung A - T) das Nukleotid ein. Im nächsten Schritt wird die Reaktionslösung D mittels des sich an D anschließenden Waschpuffers C weggewaschen und es erfolgt eine optische Detektion der an den eingebauten Nukleotiden befindlichen Fluorophoren. Dies geschieht bevorzugt mit der beschriebenen Anregungseinheit 23 und der beschriebenen Detektionseinheit 27. Nun wird mittels der in Fig. 2 nicht dargestellten Fördereinrichtung das nächste Flüssigkeitspaket Plug E in die Zellen transportiert. Plug E ist beispielsweise Enzym2, was die Terminatoren der Nukleotide entfernt. Der sich an E anschließende Waschplug C ist wieder die Kombination aus Waschpuffer und Verdrängermedium. Nun wird mittels der Pumpeinheit Plug F in die Zellen transportiert. F ist ein Gemisch aus Enzym1 und beispielsweise ddNTP "C". Wird das Array mit Plug F beaufschlagt, baut das Enzym bei einem in dem abzulesenden Strang vorhandenen "G" das Nukleotid ein (wegen der Basenpaarung G - C). Dieser zyklische Prozess wird so lange durchgeführt, bis die gewünschte Leselänge erreicht ist. Mittels einer optischen Kontrolle können die genauen Positionen der Phasengrenzen (oder anders gesagt: die Position der verschiedenen Flüssigkeitsplugs) detektiert werden.

Die mit der Detektionseinheit durchgeführte Detektion ist bevorzugt eine optische Kontrolle. Beispielsweise ist die Detektionseinheit eine Kamera, mit welcher das Durchflussarray 2 und/oder die Zirkulationsleitung 6 überwacht werden können und mit welcher die Phasengrenzen zwischen den Flüssigkeitsplugs erkannt werden können. Die wässrigen Phasen und/oder die Ölphasen können dafür mit verschiedenen Farbstoffen oder Fluorophoren eingefärbt sein (bspw. Farbstoffe HEX, FAM; Rußpartikel, Lebensmittelfarbstoffe, ...). Durch diese Positionsbestimmung kann gesteuert werden, welche Lösungen wiederverwendet werden, welche einem Abfallbehälter an der Zirkulationsleitung 6 bzw. einem Filter ab der Zirkulationsleitung 6 zugeführt werden. Ein Beispiel für die Wiederverwendung von Flüssigkeiten ist beispielsweise das Enzymgemisch Enzym1 + ddNTP "A", "G", "T", "C". Diese Reagenzien können in die jeweiligen Reservoire zurückgeführt werden, um diesen für einen erneuten Einbauzyklus entnommen zu werden. Genauso können die inerten Ölplugs in das jeweilige Reservoir zurückgeführt werden.

Somit werden mit dem beschriebenen fluidischen Netzwerk drei Ansätze, um Puffervolumina einzusparen, möglich:
1) Wiederverwendung von Volumina durch Rückführung in ihre Reservoire;
2) Verwendung von Zweiphasen-Gemischen für die Reduktion von Totvolumina und das präzise Pumpen definierter Volumina;
3) Filtrationsmöglichkeiten von Waschlösungen für die Wiederverwendung der Waschlösung.

Nachfolgend werden Ausführungsformen beschrieben, um die sequenzierenden DNA-Kolonien (Abbildung 2, B) in den Kolonien bereitzustellen/zu immobilisieren.

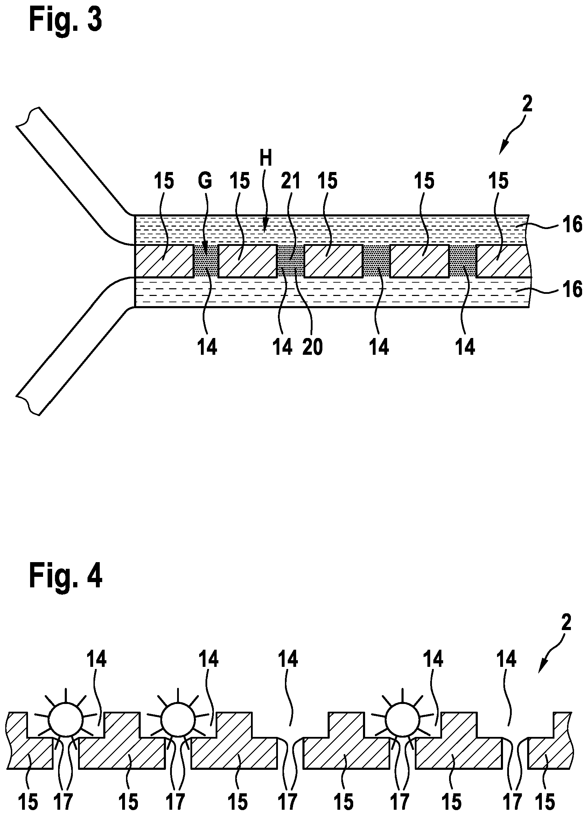

Fig. 3 zeigt einen Querschnitt des Durchflussarrays 2. Zu erkennen sind die einzelnen Reaktionsräume 14, die durch Trennstege 15 voneinander getrennt sind und in welche durch die Versorgungsleitungen 16 die jeweiligen Reaktionsflüssigkeiten eingebracht werden können. Die Reaktionsräume 14 bilden Durchflusszellen des Durchflussarrays. Der Durchmesser der Reaktionsräume 14 bzw. der Durchflusszellen liegt hier zwischen 1 und 500 µm, bevorzugter Weise zwischen 1 µm und 50 µm. In den Reaktionsräumen 14 bzw. in den Durchflusszellen können DNA-Primer vorgelagert sein ("Targeted Sequencing"), um in den Zellen Amplifikationsprodukte zu generieren, die an den Wänden der jeweiligen Reaktionsräume 14/Durchflusszellen immobilisiert werden. Vorteilhafterweise ist in jedem der Reaktionsräume 14/Durchflusszellen ein anderes Primerpärchen vorgelagert, um in den verschiedenen Zellen unterschiedliche DNA-Moleküle ablesen zu können. In ein derartiges Durchflussarray 2 wird eine Lösung eingefüllt, die mindestens die zu sequenzierenden DNA-Moleküle und eine Polymerase enthält. Diese Lösung wird in die Durchflusszellen/Reaktionsräume 14 des Durchflussarrays 2 eingefüllt. Für die nachfolgende Reaktion wird das Durchflussarray mit dem Öl "H" so versiegelt, dass die ersten Kanalanschlüsse 20 und die zweiten Kanalanschlüsse 21 (Ober- und Unterseite) der Reaktionsräume 14 mit dem Öl abgeschlossen sind. Dieser Zustand ist auch in Fig. 3 gezeigt. Nach Durchführung einer Amplifikationsreaktion (PCR, LAMP, RPA, SDA, ...) sind die entstandenen DNA-Kolonien "G" an den Wänden der jeweiligen Reaktionsräume 14 immobilisiert. Dieser Zustand ist ebenfalls in Fig. 3 gezeigt. Die aufeinander folgende Befüllung und Austausch von Lösungen in den Reaktionsräumen 14 erfolgt wie zuvor beschrieben, nämlich durch präzises Fördern der Fluide in der Zirkulationsleitung.

In Fig. 4 ist eine weitere Variante gezeigt, wie die zu sequenzierenden DNA-Kolonien in den Zellen vorhanden sein können. Hierbei ist eine neue Form eines Durchflussarrays 2 mit Reaktionsräumen 14 gezeigt, wobei Fig. 4 nur einen Ausschnitt des Durchflussarrays 2 zeigt. Insbesondere sind der erste Versorgungskanal 16 und der zweite Versorgungskanal 19 hier nicht gezeigt. Der Schwerpunkt der Fig. 4 liegt auf der Darstellung der abtrennenden Trennstege 15, welche die einzelnen Reaktionskammern 14 des Durchflussarrays 2 voneinander abtrennen. Die Reaktionsräume 14 sind hier mit Rückhaltenasen 17 ausgestattet, mit welchen Partikel 18 zurückgehalten werden können. Die DNA-Kolonien in den Reaktionskammern 14 werden hier auf den Partikeln 18 bereitgestellt. Im Rahmen eines BEAMing-Verfahrens entstehen Mikropartikel, auf deren Oberfläche die zu sequenzierenden DNA-Kolonien immobilisiert sind. Wird eine diese Partikel 18 enthaltende Lösung durch ein wie in Fig. 4 gezeigtes Durchflussarray 2 gespült, werden die Partikel 18 von den in dem Durchflussarray 2 vorhandenen Rückhaltenasen 17 fixiert/positioniert. So entsteht beispielhaft ein sequenzierfähiges Durchflussarray 2. Der Durchmesser der durch die Rückhaltnasen 17 definierten Öffnungen ist etwas kleiner als der der Partikel. Die Durchmesser der Reaktionsräume 14 liegen hier zwischen 1 nm und 100 µm. Ein solches Partikel 17 enthaltendes Durchflussarray kann mittels eines mikrofluidischen Mehrphasenansatzes - wie oben beschrieben - sequenziert werden.

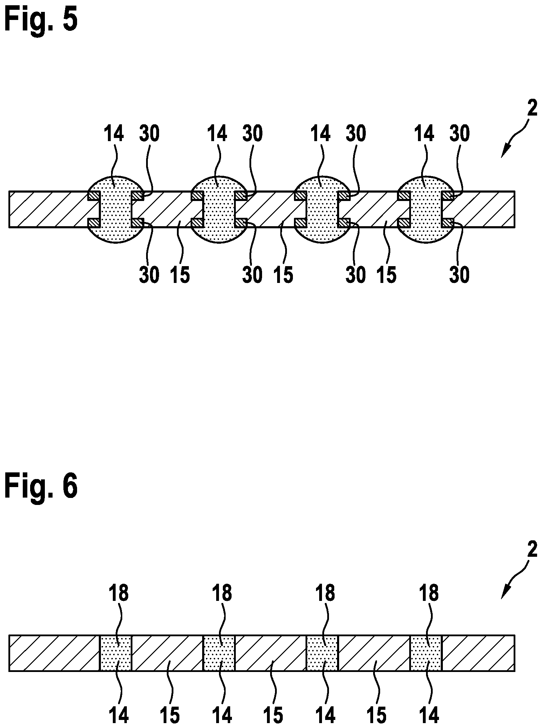

Fig. 5 zeigt eine weitere Möglichkeit, zu sequenzierende DNA-Kolonien in den Reaktionsräumen 14 eines Durchflussarray 2 zu generieren. Auch in Fig. 5 ist das Durchflussarray 2 nur ganz schematisch gezeigt. Hier sind mindestens die innen liegenden Wände der Zellen und die Randbereiche auf Ober- und/oder Unterseite mit einer hydrophilen Beschichtung 30 hydrophil beschichtet. Die hydrophile Beschichtung 30 auf Ober- und Unterseite sind in Fig. 5 oben als schwarze Punkte gezeigt. Der Rest des Arrays ist vorzugsweise hydrophob.

Das Durchflussarray 2 weist (wie bereits beschrieben) Reaktionsräume 14 mit einem Zellendurchmesser von 1 nm bis 100 µm auf. Wird ein solches Array mit einer wässrigen Phase beaufschlagt, hinterbleiben Flüssigkeitströpfchen in den hydrophilen Bereichen, wie in Fig. 5 gezeigt. Durch diese Variante kann nochmals Reaktionslösung eingespart werden und die Dichte an Zellen zusätzlich erhöht werden. Ein weiterer Vorteil ist der, dass die DNA-Kolonien im Rahmen einer Digitalen PCR generiert werden können. Hierbei wird die Anzahl an zu sequenzierenden DNA-Molekülen so eingestellt, dass diese verhältnismäßig klein ist. Wird eine Lösung, die mindestens die DNA-Moleküle und eine Polymerase enthält, in die Zellen des Arrays eingefüllt, verteilen sich die Moleküle gemäß der Poisson-Verteilung auf die Zellen (danach kommt wieder eine Amplifikationsreaktion, im Rahmen derer die Amplifikationsprodukte an den Wänden der Zellen immobilisiert werden).

Fig. 6 zeigt noch eine weitere Ausführungsvariante eines Durchflussarrays. Hier können die Zellen/Reaktionsräume 14 des Durchflussarrays mit einem gewebeartigen Material 18 bzw. einem porösen Material ausgeformt sein. Dies hat den Vorteil, dass die Befüllung der Zellen/Reaktionsräume 14 durch Kapillarkräfte unterstützt wird, die vorgesehenen Reaktionsvolumina weiter reduziert werden und ein diffusives Waschen ermöglicht wird.

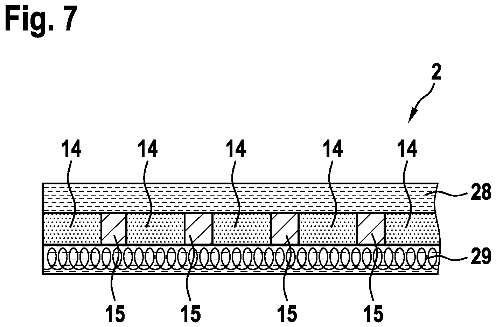

Fig. 7 verdeutlicht nochmal die Vorgänge des beschriebenen Verfahrens. In Fig. 7 ist gezeigt, dass eine Seite des Durchflussarrays während des gesamten Sequenzierprozesses (oder auch nur bei bestimmten Schritten) mit einer Ölphase 28 verschlossen sein kann. So können einzelne wässrige Phasen 29 von Reagenzien, die für eine Reaktion verwendet werden, auf der anderen Seite über das Array gespült werden. Der Waschwirkung erfolgt hier fast ausschließlich diffusiv. Dieses fluidische Prinzip ("Halb verschlossenes Array") kann auch für ein Durchflussarray, wie in Fig. 4 gezeigt, eingesetzt werden. Hierbei werden die benötigten Mengen an Waschlösung weiter reduziert.

Das Verfahren ist durch optische Inspektion eines Reaktionsträgers, im speziellen des Sequenzierbereiches und Analyse der verwendeten Prozessflüssigkeiten nachweisbar.

## Patentansprüche

1. Fluidisches Netzwerk (1) zur parallelen Durchführung einer Vielzahl von Untersuchungen von biologischen Proben, aufweisend ein Durchflussarray (2) mit einer Vielzahl von Reaktionsräumen (14), wobei die Reaktionsräume (14) jeweils einen ersten Kanalanschluss (20) und einen zweiten Kanalanschluss (21) aufweisen, wobei die ersten Kanalanschlüsse (20) an einen ersten Versorgungskanal (16) angeschlossen sind und die zweiten Kanalanschlüsse (21) an einen zweiten Versorgungskanal (19) angeschlossen sind, wobei der erste Versorgungskanal (16) und der zweite Versorgungskanal (19) mit einer Zirkulationsleitung (6) miteinander verbunden sind, wobei an der Zirkulationsleitung mindestens eine Komponente (3, 4, 5, 7) angeschlossen ist, mit welcher Versuchsreagenzien in die Reaktionsräume (14) des Durchflussarrays (2) einbringbar sind.

2. Fluidisches Netzwerk (1) nach Anspruch 1, wobei das fluidische Netzwerk (1) zur Untersuchung einer biologischen DNA-Probe und zur Erkennung von Nukleinsäuresequenzen in der DNA-Probe eingerichtet ist.

3. Fluidisches Netzwerk (1) nach Anspruch 2, wobei an die Zirkulationsleitung (6) mindestens eine Fördereinrichtung (4) angeschlossen ist, mit welcher Reagenzien über den ersten Versorgungskanal (16) und/oder über den zweiten Versorgungskanal (19) in die Reaktionsräume (14) förderbar sind.

4. Fluidisches Netzwerk (1) nach einem der vorhergehenden Ansprüche, wobei an die Zirkulationsleitung (6) mindestens ein Reservoir (5) angeschlossen ist, in welchem ein Reagenz bereitgestellt wird.

5. Fluidisches Netzwerk (1) nach einem der vorhergehenden Ansprüche, wobei an die Zirkulationsleitung (6) mindestens ein Reservoir (5) angeschlossen ist, in welchem ein Verdrängermedium bereitgestellt wird.

6. Fluidisches Netzwerk (1) nach Anspruch 4, wobei mindestens ein Reservoir (5) mit einem Ventil (3) der Zirkulationsleitung (6) zuschaltbar ist.

7. Fluidisches Netzwerk (1) nach einem der vorehrgehenden Ansprüche, wobei in der Zirkulationsleitung (6) mindestens ein Filter (7) angeordnet ist, mit welchem ein in der Zirkulationsleitung (6) bewegtes Reagenz filterbar ist.

8. Fluidisches Netzwerk (1) nach einem der vorhergehenden Ansprüche, wobei die Reaktionsräume (14) in dem Durchflussarray (2) in einer zweidimensionalen Matrix angeordnet sind.

9. Fluidisches Netzwerk (1) nach einem der vorhergehenden Ansprüche, wobei die Reaktionsräume (14) in dem Durchflussarray (2) mit Trennstegen (15) voneinander abgetrennt sind.

10. Fluidisches Netzwerk (1) nach einem der vorhergehenden Ansprüche, wobei in den Reaktionsräumen (14) Rückhaltenasen (17) vorgesehen sind, mit welchen Partikel in den Reaktionsräumen gehalten werden können.

11. Fluidisches Netzwerk (1) nach einem der vorhergehenden Ansprüche, wobei auf einer ersten Seite (22) des Durchflussarrays (2) ein Einkopplungsbereich (25) zum Einbringen einer Anregung mit einer Anregungseinheit (23) und auf einer zweiten Seite (24) des Durchflussarrays (2) ein Auslesebereich (26) zum optischen Auslesen des Durchflussarrays (2) mit einer Detektionseinheit (27) angeordnet sind.

12. Fluidisches Netzwerk (1) nach einem der vorhergehenden Ansprüche, wobei das Durchflussarray (2) als austauschbares Einlegeteil ausgeführt ist.

13. Fluidisches Netzwerk (1) nach einem der vorhergehenden Ansprüche, wobei ein Durchmesser von Reaktionsräumen (14) in dem Durchflussarray (2) zwischen 1 nm [Nanometer] und 100 µm [Mikrometer] beträgt.

14. Verfahren zur Durchführung einer Analyse einer biologischen Probe aufweisend die folgenden Schritte:
a) Bereitstellen eines Fluidischen Netzwerks (1) nach einem der vorstehenden Ansprüche,
b) Bereitstellen mindestens einer Probe in einem Durchflussarray (2) des fluidischen Netzwerks.
c) Bereitstellen von mindestens zwei verschiedenen Reagenzien in dem Fluidischen Netzwerk (1), wobei die beiden verschiedenen Reagenzien durch ein Verdrängermedium voneinander getrennt sind.
d) Beaufschlagen der Probe mit den mindestens zwei verschiedenen Reagenzien, wobei ein direkter Kontakt der Reagenzien durch das Verdrängermedium verhindert wird.

## Claims

1. Fluidic network (1) for parallel performance of a multiplicity of analyses of biological samples, comprising a flow array (2) having a multiplicity of reaction spaces (14), wherein the reaction spaces (14) each have a first channel connection (20) and a second channel connection (21), wherein the first channel connections (20) are connected to a first supply channel (16) and the second channel connections (21) are connected to a second supply channel (19), wherein the first supply channel (16) and the second supply channel (19) are connected to one another by means of a circulation line (6), wherein the circulation line has connected thereto at least one component (3, 4, 5, 7) by means of which test reagents are introducible into the reaction spaces (14) of the flow array (2).

2. Fluidic network (1) according to Claim 1, wherein the fluidic network (1) is configured for analysing a biological DNA sample and for identifying nucleic acid sequences in the DNA sample.

3. Fluidic network (1) according to Claim 2, wherein the circulation line (6) has connected thereto at least one conveyor (4) by means of which reagents are conveyable into the reaction spaces (14) via the first supply channel (16) and/or via the second supply channel (19).

4. Fluidic network (1) according to any of the preceding claims, wherein the circulation line (6) has connected thereto at least one reservoir (5) in which a reagent is provided.

5. Fluidic network (1) according to any of the preceding claims, wherein the circulation line (6) has connected thereto at least one reservoir (5) in which a displacement medium is provided.

6. Fluidic network (1) according to Claim 4, wherein at least one reservoir (5) is connectable to the circulation line (6) by means of a valve (3).

7. Fluidic network (1) according to any of the preceding claims, wherein the circulation line (6) has arranged therein at least one filter (7) by means of which a reagent moved in the circulation line (6) is filterable.

8. Fluidic network (1) according to any of the preceding claims, wherein the reaction spaces (14) in the flow array (2) are arranged in a two-dimensional matrix.

9. Fluidic network (1) according to any of the preceding claims, wherein the reaction spaces (14) in the flow array (2) are separated from one another by means of partition webs (15).

10. Fluidic network (1) according to any of the preceding claims, wherein the reaction spaces (14) have provided therein retention projections (17) by means of which particles can be held in the reaction spaces.

11. Fluidic network (1) according to any of the preceding claims, wherein a first side (22) of the flow array (2) has arranged thereon a coupling-in zone (25) for introduction of an excitation by means of an excitation unit (23) and a second side (24) of the flow array (2) has arranged thereon a reading zone (26) for optical reading of the flow array (2) by means of a detection unit (27).

12. Fluidic network (1) according to any of the preceding claims, wherein the flow array (2) is realized as an exchangeable insert component.

13. Fluidic network (1) according to any of the preceding claims, wherein a diameter of reaction spaces (14) in the flow array (2) is between 1 nm [nanometer] and 100 µm [micrometer].

14. Method for performing an analysis of a biological sample, comprising the following steps:
a) Providing a fluidic network (1) according to any of the preceding claims.
b) Providing at least one sample in a flow array (2) of the fluidic network.
c) Providing at least two different reagents in the fluidic network (1), wherein the two different reagents are separated from one another by a displacement medium.
d) Exposing the sample to the at least two different reagents, wherein direct contact between the reagents is prevented by the displacement medium.

## Revendications

1. Réseau fluidique (1) pour la réalisation parallèle d'une multitude d'analyses d'échantillons biologiques, présentant une matrice d'écoulement (2) pourvue d'une multitude de chambres de réaction (14), les chambres de réaction (14) présentant à chaque fois un premier raccord de canal (20) et un second raccord de canal (21), les premiers raccords de canal (20) étant raccordés à un premier canal d'alimentation (16) et les seconds raccords de canal (21) étant raccordés à un second canal d'alimentation(19), le premier canal d'alimentation (16) et le second canal d'alimentation (19) étant reliés l'un à l'autre à l'aide d'une conduite de circulation (6), au moins un composant (3, 4, 5, 7), qui permet d'introduire des réactifs de test dans les chambres de réaction (14) de la matrice d'écoulement (2), étant raccordé à la conduite de circulation.

2. Réseau fluidique (1) selon la revendication 1, le réseau fluidique (1) étant conçu pour l'analyse d'un échantillon biologique d'ADN et pour la détection de séquences d'acide nucléique dans l'échantillon d'ADN.

3. Réseau fluidique (1) selon la revendication 2, au moins un dispositif de transport (4), qui permet de transporter des réactifs par l'intermédiaire du premier canal d'alimentation (16) et/ou par l'intermédiaire du second canal d'alimentation (19) dans les chambres de réaction (14), étant raccordé à la conduite de circulation (6).

4. Réseau fluidique (1) selon l'une des revendications précédentes, au moins un réservoir (5), dans lequel un réactif est mis à disposition, étant raccordé à la conduite de circulation (6).

5. Réseau fluidique (1) selon l'une des revendications précédentes, au moins un réservoir (5), dans lequel un agent de déplacement est mis à disposition, étant raccordé à la conduite de circulation (6).

6. Réseau fluidique (1) selon la revendication 4, au moins un réservoir (5) pouvant être mis en circuit à l'aide d'une vanne (3) de la conduite de circulation (6).

7. Réseau fluidique (1) selon l'une des revendications précédentes, au moins un filtre (7), qui permet de filtrer un réactif en mouvement dans la conduite de circulation (6), étant agencé dans la conduite de circulation (6).

8. Réseau fluidique (1) selon l'une des revendications précédentes, les chambres de réaction (14) dans la matrice d'écoulement (2) étant agencées dans une matrice bidimensionnelle.

9. Réseau fluidique (1) selon l'une des revendications précédentes, les chambres de réaction (14) dans la matrice d'écoulement (2) étant séparées les unes des autres à l'aide de traverses de séparation (15).

10. Réseau fluidique (1) selon l'une des revendications précédentes, des nez de rétention (17), qui permettent de retenir des particules dans les chambres de réaction, étant prévus dans les chambres de réaction (14).

11. Réseau fluidique (1) selon l'une des revendications précédentes, une zone de couplage (25), destinée à introduire une excitation à l'aide d'une unité d'excitation (23), étant agencée sur un premier côté (22) de la matrice d'écoulement (2) et une zone de lecture (26), destinée à la lecture optique de la matrice d'écoulement (2) à l'aide d'une unité de détection (27), étant agencée sur un second côté (24) de la matrice d'écoulement (2).

12. Réseau fluidique (1) selon l'une des revendications précédentes, la matrice d'écoulement (2) étant réalisée sous forme d'une pièce d'insertion pouvant être remplacée.

13. Réseau fluidique (1) selon l'une des revendications précédentes, un diamètre des chambres de réaction (14) dans la matrice d'écoulement (2) étant situé entre 1 nm [nanomètre] et 100 µm [micromètres].

14. Procédé de réalisation d'une analyse d'un échantillon biologique, présentant les étapes suivantes :
a) mise à disposition d'un réseau fluidique (1) selon l'une des revendications précédentes,
b) mise à disposition d'un échantillon dans une matrice d'écoulement (2) du réseau fluidique,
c) mise à disposition d'au moins deux réactifs différents dans le réseau fluidique (1), les deux réactifs différents étant séparés l'un de l'autre par un agent de déplacement,
d) soumission de l'échantillon auxdits au moins deux réactifs différents, un contact direct des réactifs étant empêché par l'agent de déplacement.
